# EUROPÄISCHE PATENTANMELDUNG

(11) **EP 2 042 165 A1**
(43) Veröffentlichungstag der Anmeldung: **01.04.2009**
(21) Anmeldenummer: 07117480.9
(22) Anmeldetag: 28.09.2007
(51) Int. Cl.: A61K 9/48

(54) **Hot-Melt-Befüllte Weichkapseln**

(71) Anmelder: Swiss Caps Rechte und Lizenzen AG, 9533 Kirchberg (CH)
(72) Erfinder: Schwab, Ernst, 9500 Wil (CH); Plum, Georg, 78467 Konstanz (DE)
(74) Vertreter: Welch, Andreas

(57) **Zusammenfassung**

Die vorliegende Erfindung betrifft eine Weichkapsel, umfassend eine Hülle und ein Füllgut mit mindestens einem pharmakologisch oder physiologisch aktiven Stoff, **dadurch gekennzeichnet, dass** die Hülle aus durch Schmelzextrusion thermoplastisch verarbeitbarem polymerem Material aufgebaut ist und das Füllgut unterhalb von 21°C fest ist und einen Tropfpunkt von höher als 37°C aufweist.

## Beschreibung

Die vorliegende Erfindung betrifft Weichkapseln enthaltend eine bei Raumtemperatur erstarrte Füllmasse, die als heisse geschmolzenen Füllmasse in die Kapsel gefüllt wird, sowie ein Verfahren zu deren Herstellung.

Kapseln sind etablierte Darreichungsformen für Arzneimittel und Nahrungsergänzungsmittel. Sie sind als Kern-Hülle-Struktur ausgebildet, d.h. ein Inhaltsstoff beliebiger Konsistenz (der Kern, auch als Füllgut bezeichnet) ist von einer Hülle aus geeignetem Hüllenmaterial umgeben. Man unterscheidet hierbei insbesondere Hartkapseln und Weichkapseln.

Kapseln und Verfahren zu ihrer Herstellung sind hinlänglich bekannt (z.B. Stanley, J.P. "Soft gelatin capsules" in: Lachman et al. (Hrsg.) "The theory and practice of industrial pharmacy", Philadelphia, Lea & Febiger, 3rd edition (1986); Hofer et al. in: Fahrig, W.; Hofer, U. (Hrsg.) "Die Kapsel", Wiss. Verlagsgesellschaft mbH, Stuttgart; Paperback APV Band 7, 1. Aufl.,1981).

Bei Hartkapseln besteht das Hüllenmaterial aus einem eher dünnen Film (mit einer Dicke bis zu 200 µm), der aber dennoch formstabil ist. In der Regel ist die Hülle aus zwei sich ergänzenden und zusammenfügbaren Teilen ("body" und "cap") aufgebaut.

Die beiden Kapselhüllenteile werden in einem ersten Schritt aus wässrigen Lösungen von Polymeren geformt und getrocknet. In der Regel werden Polymere verwendet, die in Lösung einen Sol-Gel Übergangszustand aufweisen (Gelatine, HPMC (Hydroxypropylmethylcellulose) + Carrageenan, usw.). Bedingt durch die die Technologie der Herstellung können nur sehr eng begrenzte Zusammensetzungen von Polymerlösungen verwendet werden. Es können in der Regel keine Variationen der Zusammensetzung der Hülle vorgenommen werden, etwa um später Interaktionen mit dem Füllgut zu vermeiden, oder um die fertige Kapsel bei besonders niedrigen oder besonders hohen Feuchten stabil zu halten.

In einem zweiten Schritt erfolgt das Trennen von Ober- und Unterteil, das Einfüllen des Füllgutes in das Unterteil und das erneute zusammenstecken der beiden Hüllenteile. In der Kapsel bleibt immer ein Gas (Luft) im Innenraum der Kapsel. Bei flüssigen Füllgütern oder Füllgütern, die sich während der Lagerung verflüssigen können, muss die zwischen Hüllenober- und unterteil bestehende Lücke mit einem weiteren Film verschlossen (Banderollieren, oder Spray-Film Coating) oder mit polymerem "Leim" verklebt werden.

Als Hüllenmaterial werden üblicherweise Gelatine, Cellulosederivate, Gummen, PVA (Polyvinylalkohol), PVP (Polyvinylpyrrolidon) und andere synthetische und natürliche Polymere oder Mischungen von Polymeren mit andern Stoffen verwendet. Hartkapseln werden in der Regel mit pulverigen, oder partikulären (Pellets) Füllgütern befüllt, seltener (aber auch) mit flüssigen, pastösen Füllgütern. Um das teure zusätzliche Verschliessen oder Coating zu vermeiden, wurden insbesondere auch Füllgüter entwickelt, die in geschmolzener Form eingebracht werden, bei Lagerbedingungen erstarrt sind und bei der Lagerung nicht schmelzen und auslaufen können.

Durch Erstarrung verfestigte Schmelzen lipophiler Stoffe sowie hochviskose Suspensionen von wasserlöslichen Partikeln in lipophiler Matrix eignen sich zur Befüllung von Hartkapseln: Hartkapseln auf der Basis von Gelatine oder HPMC enthalten kaum Weichmacher und Wasser (aw= 0.5, ca. 8% Wasser für Gelatine und ca. 4% für HPMC als Grundmaterial). Demzufolge sind deren mechanischen Eigenschaften auch ziemlich tolerant gegen lipophile Stoffe und Temperaturen bis 80°C. Mit anderen Worten schmelzen oder verformen sich die Hüllen nicht bei Temperaturen von 25-60°C.

Die Befüllung von Hartkapseln mit wachsartigen Stoffen wie Fetten, Partialglyceriden, Fettsäureestern (Polyethylenglycol-Fettsäureester, Gelucire) und langkettigen Polyethylenglycolen (PEG) ist bekannt. Solche Formulierungen eignen sich vor allem für die Formulierung von retardierten, langsam freisetzenden pharmazeutischen Präparaten, oder zur Formulierung von schwer wasserlöslichen aktiven Stoffen in wasserfreien, aber wasserlöslichen Matrizes.

Bei Weichkapseln ist die Hülle in der Regel dicker (mit einer Dicke über 200 µm). Das Hüllenmaterial enthält einen Weichmacher. Bedingt durch das Herstellungsverfahren bestehen Weichkapseln in der Regel aus einteiligen Filmen, d.h. Hüllen die auch gegen nicht wässrige Flüssigkeiten dicht sind. Als Filmmaterial hat sich in der Vergangenheit vor allem Gelatine bewährt. Die Herstellung erfolgt mit Hilfe von folgenden Techniken: Tropfverfahren, Ringextrusionsverfahren, injection moulding (Spritzgussverfahren), Coinjection moulding-Verfahren oder Verschweissung zweier halbschaliger Filme (z.B. Colton-, Upjohn-, Accogel-, Norton- oder Rotary die-Prozess).

Um die notwendige Flexibilität der Filme bei der Vertropfung oder der Formung und Verschweissung sicherzustellen, bestehen die Filme aus wässrigen Lösungen enthaltend Polymer, Weichmacher und Zuschlagstoffe. Das überschüssige Wasser wird durch Trockenen der geformten und befüllten Kapsel bis zum dem für die Lagerung notwendigen Anteil entfernt.

Weichkapseln werden in der Regel mit flüssigen oder pastösen Füllgütern (Suspension) befüllt. Sie können im Prinzip auch - mit Hilfe einer speziellen Dosiervorrichtung - mit Partikeln (Granulat oder Pellets) suspendiert in flüssigen Phasen oder mit pulverigen oder partikulären (Pellets) Füllgütern (ohne flüssige Phase) befüllt werden. Bevorzugt, da kaum Wechselwirkungen zwischen hydrophiler Hülle und Füllgut eintreten, sind liphophile flüssige Füllstoffe allein oder als Trägermatrix für eine Suspension von kristallinen wasserlöslichen Stoffen.

Die Verfahrensparameter (Temperatur, Druck, Zeit) beim Einbringen von Füllgütern in Weichkapseln sind bedingt bzw. limitiert durch die Bedingungen, welche für die gleichzeitige Verformung und Versiegelung der zwei Hüllenteile angewandt werden müssen. So ist bei der traditionellen Weichgelatinekapselherstellung aus wässrigen Gelatinelösungen (nach dem Rotary Die Verfahren, vgl. Bauer. in: Fahrig, W.; Hofer, U. (Hrsg.) "Die Kapsel", Wiss. Verlagsgesellschaft mbH, Stuttgart; Paperback APV Band 7, 1983, S. 70) bedingt durch den Gel-Sol-Übergang der wässrigen Gelatinebänder (bei ca. 40-45°C) die bei der Kapselherstellung tolerierbare Temperatur auf ca. 35°C beschränkt (US-6,352,717, Sp. 1, Z. 60-67). Analog ist bei Verwendung von Stärke/Carrageen-Wasser-Lösungen, ebenfalls bedingt durch den Sol-Gel-Übergang (bei ca. 50-70°C), die bei der Befüllung des Füllgutes anwendbare Temperatur auf maximal ca. 50°C beschränkt. Ausserdem ist das in den Filmen enthaltene Wasser oft problematisch für die Füllgüter.

Für spezielle Zusammensetzungen kann es gelingen, die Füllgutschmelzen durch Scherviskosität am Erstarren zu hindern, oder eine kurzfristige Unterkühlung der Schmelze vor Verbringung in die Weichgelatinekapsel durch spezielle Abfüllvorrichtungen zu erreichen. Derartige Methoden sind in der US 6352717 offenbart.

Das Einbringen von geschmolzenen Füllgütern zwischen zwei einhüllende Bänder mittels Extrusion / Kalandrierung wurde in der EP-0 799 012 A1 für die Herstellung von Tabletten beschrieben. Diese Technik ist aber insbesondere bei der Kalandrierung von konzentrischen Strängen (Füllgut innen, von äusserer Hülle umgeben) nicht als die Herstellung kapselförmiger Körper zu bezeichne, da die Menge an Füllgut und die Filmdicke sich am Rand der Kalanderform auf Null verjüngt und daher keineswegs eine relativ gleichförmige Hüllendicke rund um den Formkörper zu erreichen ist. Die so entstehenden Gräte verunmöglichen das Einnehmen, und die Verdünnung der Hülle erschwert die Kontrolle der galenischen Eigenschaften (zum Beispiel bei magensaftresistenter Ausprägung der Hülle).

Die Technologie der Schmelzextrusion (melt extrusion) und Formung mit Spritzguss (injection moulding) ist ebenfalls hinreichend bekannt und hat insbesondere die Vorteile, feste erstarrte Schmelzen, thermoplastische Polymere und Glase enthaltend molekulardispers verteilte Wirkstoffe als Füllgut einsetzen zu können. Das anschliessende Überziehen solcher Körper durch Tauchen oder Spray Coating führt zu Produkten, die im technologischen Sinne ebenfalls nicht als Kapsel zu bezeichnen sind.

Die Herstellung von Weichkapseln mit festen Füllgütern war somit bislang limitiert: Die Befüllung mit Partikeln oder Pulvern erfordert den Einsatz spezieller Dosiervorrichtungen und macht damit den Umbau der Kapselherstellungsvorrichtung notwendig. Die Befüllung von Weichgelatinekapseln mit Schmelzen ist hingegen aus herstellungstechnischen Gründen auf Füllgüter beschränkt, die bei Temperaturen von bis zu 35°C (im Fall von Stärke/Carrageen-Wasser-Lösungen zur Filmherstellung bei bis zu 50°C) schmelzen. Andererseits wären Weichkapseln mit festem Füllgut aus den vorstehend diskutierten Gründen gegenüber Hartkapseln bevorzugt (beispielsweise die Bereitstellung von Hüllen mit unterschiedlichen mechanisch-galenischen Eigenschaften sowie die Möglichkeit der Minimierung von Wechselwirkungen zwischen Hülle und Füllgut).

Es war daher die Aufgabe der vorliegenden Erfindung, Weichkapseln mit schmelzbaren Füllgut unabhängig von dessen Schmelztemperatur bereitzustellen.

Diese Aufgabe wird gemäss der vorliegenden Erfindung gelöst durch eine Weichkapsel, umfassend eine Hülle und ein Füllgut mit mindestens einem pharmakologisch oder physiologisch aktiven Stoff, dadurch gekennzeichnet, dass die Hülle aus durch Schmelzextrusion thermoplastisch verarbeitbarem polymerem Material aufgebaut ist und das Füllgut unterhalb von 21 °C fest ist und einen Tropfpunkt von höher als 37°C aufweist.

Mit der vorliegenden Erfindung gelingt es, die aus der Herstellung von flüssigen, pastösen und mit Schmelzen befüllten Hartkapseln bekannten galenischen Vorteile (insbesondere Retardierung und molekulare Dispergierbarkeit) bei der Applikation pharmazeutischer Stoffe auf die Herstellung von Weichkapseln zu übertragen und damit Vorteile der "meltextrusion" Technik für die Herstellung wasserarmer Hüllen zu kombinieren, ohne den Aufwand von Banderolierung oder Coating wie bei der Herstellung von Hartkapseln hinnehmen zu müssen.

Mit der vorliegenden Erfindung werden eine Reihe von technologischen Hindernissen überwunden, die im bisherigen Stand der Technik noch nicht gelöst werden konnten:
- Bereitstellung eines hitzetoleranten, plastisch verarbeitbaren, siegelbaren, wasserlöslichen Hüllenmaterials.
- Die Verträglichkeit der Hülle und des Wirkstoffes (Füllgutes) bei verschiedenen Temperaturen, Druck- und Wasserkonzentrationen wird erreicht.
- Die unterschiedlichen Wärmeausdehnungskoeffizienten von Füllgut und Hülle beim Abkühlen der Kapsel können durch Zusätze kristalliner Art kompensiert werden.
- Vermeidung von Ausgasung und der Bildung von Gasblasen im Füllgut durch Erhitzen des Füllgutes durch vorgängige Warme- und Vakuumbehandlung und zwischen Füllgut und Hülle.
- Schutz der Wirkstoffe vor schädlicher Wärme bei Ansatz/ Herstellung.
- Variable Einstellung des Freisetzungsverhaltens (Magensaftresistenz der Hülle, sofortige oder retardierte Freisetzung des Wirkstoffes).
- Vermeidung der Rekristallisation von API oder Füllgut-Matrix aus glasigen oder teilkristallinen Systemen bei Temperaturen von mehr als 1-25°C über dem Tg (Glasübergangspunkt), was vor allem von Bedeutung ist, wenn der Tg nahe bei Null ist.

Gegenüber dem Stand der Technik ergeben sich gemäss der vorliegenden Erfindung folgende Vorteile:
- Die erfindungsgemässe Weichkapsel ist mechanisch flexibel und stabiler als Hartkapseln. Die Kapseln können nicht auslaufen.
- Die erfindungsgemässen Weichkapselfüllgüter können leicht so formuliert werden, dass sie retardiert freigesetzt werden.
- Die mechanischen/chemischen Eigenschaften der Hülle der erfindungsgemässen Weichkapsel können leicht an verschiedene Eigenschaften des Füllgutes oder des Lagerklimas angepasst werden.
- Die Kapselhülle kann aus diversen thermoplastischen Materialien geformt werden, insbesondere auch aus magensaftresistenten Polymeren. Ein zusätzliches Coating entfällt bei den erfindungsgemässen Weichkapseln.
- Die Kapselhülle enthält während der Formung und der Lagerung der Kapsel kaum Wasser (stark erniedrigter Dampfdruck). Die Füllgutzusammensetzungen verändern sich deshalb kaum durch Wasser- oder Weichmacherübertritt.
- Die Schmelztemperatur des Füllgutes kann praktisch unbegrenzt nach oben gewählt werden.
- Es ist möglich, nicht nur Füllgutmatrizes auf der Basis von Polymeren, sondern auch auf Basis von schnell wasserlöslichen, kleinen Molekülen, die schmelzen und glasig kristallin oder amorph erstarren, zu verwenden.
- Es ist möglich, die Interaktion von Hülle und Füllgut zu eliminieren, da mit zunehmender Festigkeit und zunehmender Einheitlichkeit der Phasen des Füllgutes insbesondere auch der Austausch von Weichmacher oder andern kleinen Molekülen (Diffusion) abnimmt.
- Die Molekulardispersität von Füllgütern kann mit zunehmender Festigkeit und zunehmender Einheitlichkeit der Phasen erhalten werden. Die Rekristallisation des Wirkstoffes wird verhindert.

Die erfindungsgemässen Weichkapseln zeichnen sich durch eine Kombination eines speziellen Hüllmaterials und eines speziellen Füllgutes aus.

Das Hüllmaterial ist aus durch Schmelzextrusion thermoplastisch verarbeitbarem polymerem Material aufgebaut. Unter Schmelzextrusion im Sinne der vorliegenden Erfindung ist eine Herstellung, Verformung und Verschweissung einer thermoplastischen Masse bei Drücken von mehr als 1 bar (vorzugsweise mehr als 10 bar, noch bevorzugter mehr als 100 bar) und Temperaturen von mehr als 60°C, bevorzugter mehr als 80°C, noch bevorzugter mehr als 100 °C zu verstehen.

Bei der Schmelzextrusion wird ein Band aus dem entsprechenden Material geformt, indem das Material durch eine Breitschlitzdüse oder mit Hilfe von Blasverfahren in Filmform bei Viskositäten gebracht wird, die eine Verarbeitung unter Ausnutzung der Schwerkraft (Casting) nicht erlauben. Bei der Schmelzextrusion müssen daher notwendigerweise die Temperatur (um mindestens 20-50°C) sowie der Druck (auf mehr als 10 oder sogar mehr als 100 bar) gegenüber Umgebungsbedingungen erhöht werden. Gegebenenfalls kann dem thermoplastisch verarbeitbarem Material Weichmacher und/oder Wasser sowie gängige Additive wie beispielsweise Gleitmittel zugegeben werden.

Ein exemplarisches, erfindungsgemäss verwendbares Schmelzextrusionsverfahren ist in der EP-1 103 254 A1 beschrieben. Die Schmelzextrusion kann in einem Doppelschneckenextruder durchgeführt werden, wie er in der EP-1 103 254 A1 (Ausführungsbeispiel, Fig. 4) im Detail beschrieben ist. Auf die entsprechende Offenbarung der EP-1 103 254 A1 wird ausdrücklich Bezug genommen.

Erfindungsgemäss kann das durch Schmelzextrusion thermoplastisch verarbeitbare polymere Material ausgewählt werden aus der Gruppe bestehend aus Polyvinylpyrrolidon, Hydroxypropylcellulose, Hydroxypropylmethylcellulsose, Polyvinylalkohol, Polyvinylalkoholacetalen, Polethylenglycol-Polyvinylalkohol-Pfropfpolymer, Carbopol, Polymer aus Butylmethacrylat, 2-dimethylaminoethylmethacrylat und Methylmethacrylat im Verhältnis 1 : 2 : 1 , Polyethylacrylat, Methylmethacrylat, Polymethacrylsäure, Ethylacrylat, Polyethylacrylat, Methylmethacrylat, Trimethylammoniumethylmethacrylatchlorid, Hydroxypropylmethylcelluloseacetatsuccinat, Polyox, Stärke, Polymilchsäure, Polymilchsäure-coglycolid, Gelatine, Carrageenan, Casein, Gluten und deren Mischungen.

Beispiele für erfindungsgemäss geeignete Materialien sind die in der EP-1 103 254 A1 offenbarten stärkehaltigen Massen sowie die in der EP-1 586 436 A1 offenbarten Hüllmaterialien, wobei im letzteren Fall Hüllmaterialien auf der Basis von PVACL bevorzugt sind.

Die beispielsweise in der EP-1 103 254 A1 beschriebenen stärkehaltigen Massen sind homogenisierte, Stärke enthaltende Massen, enthaltend vorzugsweise mindestens 45 Gew.-% einer amorphen Stärke mit einem Amylopektingehalt von grösser oder gleich 50 Gew.-% bezogen auf das Gewicht der wasserfreien Stärke, Wasser, mindestens einen organischen Weichmacher in einem Anteil von mindestens 12 Gew.-% bezogen auf das Gewicht der wasserfreien Stärke, wobei der Staudinger-Index der homogenisierten Masse mindestens 40 ml/g, bevorzugt mindestens 50 ml/g und noch bevorzugter mindestens 60 ml/g beträgt. Erfindungsgemäss soll der Staudinger-Index in derselben Weise wie in der EP-1 103 254 A1 definiert verstanden werden. Auf die entsprechende Offenbarung der EP-1 103 254 A1 wird ausdrücklich Bezug genommen.

Unter dem Begriff Stärke sollen native Stärken, sowie physikalisch und/oder chemisch modifizierte Stärken verstanden werden. Beispielhafte Weichmacher besitzen einen Löslichkeitsparameter von gleich oder > 16,3 (MPa)^{1/2} aufweisen. Die Weichmacher können ausgewählt werden aus der Gruppe bestehend aus Polyalkoholen, organischen Säuren, Aminen, Säureamiden und Sulfoxiden. Bevorzugt sind Polyalkohole. Als der geeignetste Weichmacher hat sich Glycerin erwiesen. In einer bevorzugten Ausführungsform liegt der Gehalt an organischen Weichmachern im Bereich von 30 Gew.% bis 50 Gew.%, bevorzugt in einem Bereich von 38 Gew.% bis 45 Gew.%.

In einer weiteren Ausführungsform enthält die Masse zusätzlich ein Gleit- und Entformmittel, welches ausgewählt ist aus der Gruppe bestehend aus Lecithinen, Mono-, Di-, und Triglyceriden von Speisefettsäuren, Zuckerester der Speisefettsäuren und Speisefettsäuren.

In einer weiteren Ausführungsform kann die Masse zusätzlich noch mindestens einen Zuschlagstoff in einem Gewichtsbereich von 3,5 Gew.% bis 15 Gew.% bezogen auf das Gesamtgewicht der Masse enthalten, bevorzugt von 5 Gew.% bis 8 Gew.%. Der Zuschlagstoff richtet sich nach den erforderlichen Eigenschaften des aus der homogenisierten Masse erzeugten Formkörpers und wird ausgewählt aus der Gruppe bestehend aus Carbonaten und/oder Hydrogencarbonaten der Alkali- und/oder Erdalkaliionen, weiteren Zerfallshilfen, Farbstoffen, Konservierungsmittel, Antioxidantien, physikalisch und/oder chemisch modifizierten Biopolymeren, insbesondere Polysaccaride und pflanzlichen Polypeptiden. Bevorzugt werden als Zerfallsmittel für einteilige Kapselhülle Kalziumcarbonat und Amylasen eingesetzt.

Alternative verwendbare Materialien sind in der EP-0 090 600 A1 beschrieben. Auf die entsprechende Offenbarung der EP-0 090 600 A1 wird ausdrücklich Bezug genommen.

Erfindungsgemäss können auch die in der EP-1 586 436 A1 beschriebenen thermoplastischen Polymer eingesetzt werden, die ausgewählt sind aus der Gruppe bestehend aus Polyvinylalkohol, Celluloseether, Polycaprolacton, Polyamiden, Polyacrylsäure, Polyvinylpyrrolidon, Polymilchsäure oder Polyvinylalkoholacetalen (PVACL), Derivaten oder Gemischen derselben. Auf die entsprechende Offenbarung der EP-1 586 436 A1 wird ausdrücklich Bezug genommen.

Erfindungsgemäss bevorzugt besteht die Hülle aus einem thermoplastischen, bei 37°C wasserlöslichen Polymer.

Erfindungsgemäss weiterhin bevorzugt besteht die Hülle aus magensaft-resistenten oder pH-abhängig sich lösenden Polymeren.

Aus diesen Materialien können durch Schmelzextrusion Filme erzeugt werden, welche in üblichen Weichkapsel-Herstellungsverfahren wie beispielsweise dem Rotary-Die-Verfahren zu Weichkapseln verarbeitet werden können. Vorzugsweise werden die beiden Schritte (Schmelzextrusion und Verkapselung) in-line (d.h. kontinuierlich) durchgeführt.

Das Rotary-Die-Verfahren ist allgemein bekannt und beispielsweise in der EP-1 103 254 A1 beschrieben. Auf die entsprechende Offenbarung der EP-1 103 254 A1 wird ausdrücklich Bezug genommen.

Gemäss der vorliegenden Erfindung erfolgt das Einbringen des Füllgutes im Kapselformungsschritt (z.B. der Rotary-Die-Methode) ebenfalls bei den erhöhten Temperaturen, die im vorhergehenden Schritt der Schmelzextrusion eingesetzt werden. Das anschliessende Verschweissen der Filme zur Kapselbildung erfolgt ebenfalls bei den für die Schmelzextrusion angegebenen erhöhten Temperatur- und Druckbedingungen. Erfindungsgemäss ist in der Regel ein Druck anzulegen, welcher den Druck bei einem herkömmlichen Rotary-Die-Verfahren um den Faktor 10 übersteigt. Erfindungsgemäss bevorzugt sind hierbei Drücke, welche den Druck bei einem herkömmlichen Rotary-Die-Verfahren um den Faktor 15 bis 40 übersteigen, d.h. etwa im Druckbereich von 225 bis 600 bar liegen.

Erfindungsgemäss besonders bevorzugt sind thermoplastische Polymere verwendbar, deren Viskositätskurven bei einer Temperatur unterhalb der Liquidus-Schmeiztemperatur im Bereich von 100 bis 750 bar eine Steigung DELTA eta / DELTA P von mindestens -30 Pas/bar und von maximal -100 Pas/bar aufweisen. Vorzugsweise erfüllen die Polymere diese Eigenschaft bei einer Temperatur im Bereich von 50 - 180 °C.

Erfindungsgemäss bevorzugt wird die Kapsel nach der Herstellung schockgekühlt, um Füllgut glasig zu machen. Dies erfolgt vorzugsweise durch kalte Gase (Stickstoff, Luft, CO₂) oder durch ein durch kaltes Bad aus mit der Hülle verträglichen Flüssigkeiten.

Die erfindungsgemäss verwendeten Hüllmaterialien haben sich überraschend als geeignet zur Herstellung von Weichkapseln erweisen, welche mit aufgeschmolzenem Füllgut befüllt werden. Insbesondere können die erfindungsgemäss verwendeten Hüllmaterialien während der Kapselherstellung deutlich höheren Temperaturen ausgesetzt werden als die bisher vornehmlich eingesetzten Materialien auf Gelatinebasis. Somit können die erfindungsgemässen Weichkapseln mit Füllgütern befüllt werden, welche einen Tropfpunkt von höher als 37°C aufweisen. Dabei kann das Füllgut bei ähnlichen Temperaturen wie vorstehend für die Schmelzextrusion angegeben Temperaturen eingebracht werden. Ein thermisch isolierter Füllkeil (wie er beispielsweise in der EP-1 216 680 A1 offenbart ist) in der Rotary-Die-Apparatur ist hierbei von Vorteil, wobei das Füllgut in diesem Fall vorzugsweise bei tieferer Temperatur als der Verkapselungstemperatur dosiert wird.

Die erfindungsgemässen Füllgüter zeigen bei Kontakt mit Wasser eine ausreichende potentielle Bioverfügbarkeit. Unter "potentiell ausreichender Bioverfügbarkeit" wird eine der pharmakologischen Wirkung und den physiologischen Gegebenheiten angepasstes sofortiges oder verzögertes molekulardisperses Angebot des API im Magen-Darmtrakt verstanden. Bei guter Wasserlöslichkeit des Stoffes sollen mindestens 80% des API in der geforderten Zeit in den Flüssigkeiten des Magen-Darmtraktes gelöst sein, was als Simulation mit der sogenannten Freisetzung gemessen werden kann. Für schlecht wasserlösliche oder lipophile, mit Wasser nicht mischbare API wird darunter die Emulgierung von mindestens 80% des API in Form von Tröpfchen, kolloidalen oder anderen komplizierten Strukturen mit einer Oberfläche von mindestens dem 1000-fachen der ursprünglichen "Kapselfüllgutoberfläche" verstanden.

Der Übergang vom "festen" in den "plastischen" oder "flüssigen" Zustand kann bei niedermolekularen und kristallisierenden Substanzen mit dem sogenannten Schmelzpunkt (Smp.) beschrieben werden. Als Schmelztemperatur bezeichnet man die Temperatur, bei der ein Stoff schmilzt, das heißt vom festen in den flüssigen Aggregatzustand übergeht. Die Schmelztemperatur ist abhängig vom Stoff, im Gegensatz zur Siedetemperatur aber nur sehr wenig vom Druck (Schmelzdruck) abhängig. Schmelztemperatur und Schmelzdruck werden zusammen als Schmelzpunkt bezeichnet, wobei dieser den Zustand eines Reinstoffes beschreibt und Teil der Schmelzkurve im Phasendiagramm des Stoffes ist. Für reine Stoffe ist der Schmelzpunkt identisch mit dem Gefrierpunkt und bleibt während des gesamten Schmelzvorganges konstant.
Für Polymere, amorphe, oder glasige Stoffe ist die Übergangstemperatur oder Glastemperatur charakteristisch. Die Glasübergangs- oder Erweichungstemperatur (Tg) ist die Temperatur, bei der ein Glas die größte Änderung der Verformungsfähigkeit aufweist. Dieser so genannte Glasübergang trennt den unterhalb liegenden spröden energieelastischen Bereich (=Glasbereich) vom oberhalb liegenden weichen entropieelastischen Bereich (=gummielastischer Bereich). Der Übergang in den Fließbereich des amorphen Kunststoffs ist fließend. Teilkristalline Kunststoffe besitzen sowohl eine Glasübergangstemperatur, unterhalb derer die amorphe Phase 'einfriert' (einhergehend mit Versprödung), als auch eine Schmelztemperatur, bei der sich die kristalline Phase auflöst. Die Schmelztemperatur trennt den entropieelastischen Bereich deutlich vom Fließbereich ab. Kristalline Kunststoffe weisen im Gegensatz dazu nur eine Schmelztemperatur auf.

Für Gemenge und Gemische aller Arten eignet sich zur Beschreibung des Verhaltens am besten der sogenannte Tropfpunkt. Dies ist die Temperatur, bei welcher das Füllgut unter Einfluss der Schwerkraft tropft (z.B. Methode USP <741> class III). Dieser Tropfpunkt ist unabhängig davon, wann die "erste" oder "letzte" Komponente "flüssig" ist, sondern richtet sich nur nach dem "mittleren" Verhalten.

Die Rotary-Die-Weichkapseltechnik dosiert das Füllgut mittels Kolbenpumpen. Es daher notwendig, dass das Füllgut bei der Verarbeitungstemperatur pumpbar ist. Das viskoelastische Verhalten eines Füllgutes muss einer Viskosität von kleiner als 50 Pas, bevorzugter kleiner als 10 Pas, noch bevorzugter kleiner als 5 Pas entsprechen. Unerheblich ist, ob die Füllgut-Mischung batchweise durch Schmelzen, Mischen, Erkalten und erneutes Aufwärmen, oder kontinuierlich direkt durch Schmelzen, Mischen und Einbringen in die Kapsel erfolgt. Ebenso lassen sich für den Prozess konventionell die Herstellung im beheizten Mischer oder mittels Extruder sinngemäss verwenden.

Erfindungsgemäss bevorzugt wird das Füllgut unmittelbar vor der Verkapselung kontinuierlich aus den entsprechenden Ausgangskomponenten durch Schmelzen und Mischen hergestellt. Vorzugsweise wird mindestens ein Wirkstoff in fester Form kontinuierlich in die Füllgut-Schmelze dosiert und gelöst oder eingemahlen. Besonders bevorzugt wird der aktive Stoff in mikropartikulärer Form der Schmelze zugesetzt. Erfindungsgemäss besonders bevorzugt wird das Füllgut kontinuierlich mit Hilfe eines Extruders zubereitet.

Überraschenderweise hat sich gezeigt, dass für die meisten gebräuchlichen pharmazeutischen, "nutritional" Wirkstoffe (Vitamine, Mineralstoffe etc) (hier generell API genannt) das Temperatur-Zeitprofil, welches sich aus der Anwendung der Rotary-Die-Verkapselung bei höheren Temperaturen ergibt, ohne negative Einwirkungen bleibt. Dies insbesondere, wenn
a) die einzelnen Rohstoffkomponenten unter Vakuum bzw. Schutzgas aufgeschmolzen wurden
b) eine Mischung der Komponenten im letzten Moment vor der Einbringung in die Kapsel erfolgt (insbesondere geeignet ist eine kontinuierliches Aufschmelz-, Misch- und Engasungsverfahren wie in einem Ein- oder Zweiwellenextruder)
c) die Verbringung in die Kapseln bei tiefstmöglicher Temperatur durch eine Füllgutleitung durch einen Füllkeil erfolgt, welche von der Erwärmung des thermoplastischen Hüllenmaterials isoliert ist
d) eine schnelle Abkühlung der hergestellten Kapsel erfolgt
e) das thermoplstische Hüllenmaterial tolerant gegenüber Erweichungs- und Schockkühlungszyklen ist
f) Wasserdampfdruck bzw. Wasserkonzentration bei den Vorgängen der Verkapselung und Kühlung beachtet wird (wasserarme Prozessführung).

Ebenso überraschend hat sich gezeigt, dass bei geeigneter Ausgestaltung von bei etwa 21°C bis 30°C festen Füllgütern, die bei Temperaturen von vorzugsweise über 48°C pumpbar und in Weichkapseln mit geeigneten Hüllenmaterialien und geeignetem Form-Verkapselungsverfahren füllbar sind, durchaus eine normale Freisetzung erreicht werden kann. Unter einer normalen Freisetzung (weder schnell (innerhalb von Minuten) noch retardiert (innerhalb von Stunden)) ist typischerweise eine Freisetzung von mehr als 80% des API innerhalb von 30 bis 60 Minuten zu verstehen. Eine solche Zubereitung umfasst den API in einer festen lipophilen selbstemulgierenden Zubereitung (SSEP Feste selbstemulgierende Phase) (solid self emulsifying phase)

Als besonders geeignet hat sich das Zumischen einer weiteren festen (polymer, kristallinen oder amorph) hydrophilen Phase erwiesen (HSP), welche bei Abfülltemperatur der Zubereitung in der selbstemulgierenden Phase (SSEP) selbst im wesentlichen unlöslich ist. Diese kann ausgewählt werden aus kristallinen oder glasigen wasserlöslichen Stoffen oder wasserunlöslichen polymeren (faserigen) Stoffen, welche in einer Art Dochtwirkung Wasser in die Zubereitung bringen oder durch schnelles Lösen, Erosion und Verteilung von SSEP Bruchstücken erlauben.

In einer besonders vorteilhaften Zubereitung besteht der Träger für den API direkt aus solchen wasserlöslichen amorphen oder teilkristallinen Schmelzen dieser Stoffe.

Als Matrix eignen sich Stoffe, die allein oder im Gemenge einen Tropfpunkt mehr als 40°C haben und pharmazeutisch oder als Lebensmittelzusatzstoff unbedenklich für die orale Aufnahme sind.

Exemplarisch seien hier genannt:
□ "weiche Schmelzmatrix": zwischen 30 und 90°C schmelzende oder erweichende, wachsartige, insbesondere auch in Wasser sich lösende oder emulgierende Stoffe: Poloxamer (Polyoxyethylen-polyoxypropylen-Blockpolymere), Sorbitanester (Span), Polyethylen-sorbitan-fettsäureester (z.B. Tween=Polysorbat 65), pflanzliche und tierische Fette (z.B. Mono- oder Di- oder Triglyceride, z.B. hydriertes Baumwollsamenöl), pflanzliche und synthetische Wachse (z. B. Bienenwachs, mikrokristalline Wachse), Tocopherolester (z.B. Vitamin E succinat, Vitamin E TPGS), Polyole (Polyethylenglycol >1500), Sucrose-Fetttsäureester (z.B.Sucrosepalmitate), Propylenglycolfettsäureester (z.B. Propylenglycolpalmitostearat), Ethylenglycolfettsäurester (z.B. Ethylenglycolpalmitostearat), Polyglycerinfettsäureester (z.B. Decaglycerin-distearat), Essigsäure-, Zitronensäure-, Milchsäure und Weinsäurefettsäureester (z.B. Imwitor 2020).
□ Wasserlösliche kristalline oder glasige Stoffe, im wesentlichen wasserfrei oder Wasser bis zur Gebrauchs-Gleichgewichtsfeuchte (aw<0.65) enthaltend: Isomalt, Sorbitol, Maltitol, Erythritol, Mannitol, Xylitol, hydrierte Hydrolyseprodukte von Stärke (Polyole und Polyol-ether), Maltodextrin, Dextrin, lösliche Stärke, Saccharose, Glucose, Fructose usw., und deren Gemische untereinander.
□ Wasserlösliche amphotere Polymere zur Verdickung: PVP (Polyvinylpyrrolidon), Acryl-oder Methacryl-Polymere (Eudragit), PEG-PVA-Pfropfpolymere (Kollicoat),
□ Lösungsmittel/Weichmacher: Glycerin, Propylenglycol, Ethyldiglycol, Salze (NaCl), Natriumsulfat, Calciumsulfat
□ Wasserunlösliche und polymere Zuschlagstoffe: Mikrokristalline Cellulose, Quervernetztes Polyvinylpyrrolidon, quervernetzte Natrium Carboxymethylcellulose, unlösliche (native) Stärken, Cyclodextrine usw.

Erfindungsgemäss kann das Füllgut folgende exemplarisch, nicht ausschliesslich aufgezählten Wirkstoffe enthalten:
□ selbst schmelzbare API: Fettsäuren, Vitamin E-succinat, Vitamin-E-nicotinat, Carotinoide-Fettsäureeste (z.B. Lutein ester), Phytosterole (Smp 80-140°C), Phytosterolester (Smp 50°) Lecithine (Phosphatidylcholin), Propranolol (Smp 96°C), Chlorathydrate (Smp 57°C), Chloramphenicolpalmitat (Smp 90°C), Cloforex (Smp 53°C), Cyclandelat (Smp 53°C) usw.

In Schmelz-Matrix eingebettete API:
□ API, welche in SSEP unter Erwärmen löslich oder dispergierbar sind, in der Matrix nicht oder nur beschränkt kristallisieren: Zum Beispiel Ibuprofen, Diclophenac Natrium, Acyclovir
□ API komplexer Zusammensetzung, wobei gewisse Fraktionen gelöst, andere nur suspendiert sind. Hier insbesondere Pflanzenextrakte verschiedenster Herkunft und Aufkonzentrierung zu nennen, welche trägerhältig oder trägerlos, spissum oder trocken vorliegen können.

Die erfindungsgemässen Weichkapseln können übliche Zusatzstoffe enthalten. Beispielsweise sind inerte kristalline Zuschlagstoffe wie Calciumtriphosphat geeignet, die Differenz der Wärmausdehnung der geschmolzenen Füllmatrix gegenüber der thermoplstischen Mischung der Hülle während des Erstarrungsvorgangs zu reduzieren, so dass es nicht zu einer partiellen Trennung der Kapselhülle von Inhalt kommt.

Unter Extrakten sind erfindungsgemäss alle Auszüge aus Pflanzenmaterial irgendwelcher Pflanzenorgane unter teilweiser Weglassung von primären Pflanzeninhaltsstoffen wie Cellulose, Sacchariden, Proteinen oder Triglyceriden zu verstehen. Der Zweck dieser Auszüge besteht in der Anreicherung bestimmter sekundärer Pflanzeninhaltsstoffe, insbesondere solchen mit erwünschten pharmakologischen Eigenschaften. Dem Fachmann ist die Zerkleinerung der Pflanzenmaterials, der Aufschluss der Pflanzenzellen durch Mahlen, Erhitzen, Ultraschallbehandlung, sowie das Extrahieren, Anreichern durch Verdampfen des Lösemittels und Reinigen hinreichend bekannt.

Gebrauchsgleichgewichtsfeuchte ist erfindungsgemäss die Produkt-Feuchte, welche sich einstellt, wenn die Kapsel durch Stehenlassen in ein Gleichgewicht mit der Umgebungsfeuchte (Luft) gebracht wird (mittlere kinetische Feuchte Klimazone I: ca. 45%RH, Produktgleichgewichtsfeuchte aw=0.45, absoluter Wassergehalt abhängig von der Sorption des Produktes).

Die Freisetzungseigenschaften der Weichkapseln werden in der vorliegenden Erfindung gemäss USP Methode <724> bestimmt.

Unter "Molekulardispers" wird gemäss der vorliegenden Erfindung eine Lösung oder lösungsähnliche bis kolloidale Verteilung des API verstanden, welche im wesentlichen frei von makroskopisch feststellbaren Konzentrationshäufungen des API in kristalliner oder amorpher Form ist. Die molekulardisperse Phase kann flüssig (Lösung), halbfest (fest und flüssig gemeinsam vorliegend) oder fest (Glas oder teilkristallin/kristallin) sein.

Die erfindungsgemässen Weichkapseln können für alle herkömmlichen Zwecke eingesetzt werden, für welche Weichkapseln zur Anwendung gelangen. Beispielhaft seien die orale, rektale oder vaginale Applikation genannt, wobei die Kapsel als Nahrungsergänzungsmittel, Pharmazeutisches Produkt, Medizinal-Produkt oder für kosmetische oder technische Zwecke appliziert werden kann.

Die vorliegende Erfindung wird nachstehend anhand von nicht einschränkenden Beispielen und Figuren näher erläutert. Es zeigen:
- Fig. 1:: Das Temperaturprofil einer Rotary-Die-Verkapselung für herkömmliche Gelatinekapseln und für die erfindungsgemässen Weichkapseln
- Fig. 2:: Eine erfindungsgemässe Ausführungsform einer Weichkapsel mit der durch Zusatz von HSP erreichten Dochtwirkung
- Fig. 3: Die Tröpfchenverteilung nach Freisetzung der Füllgutes aus einer Weichkapsel gemäss Beispiel 2
- Fig. 4: Die Freisetzungsgeschwindigkeit von Diclofenac aus einer Weichkapsel gemäss Beispiel 4
- Fig. 5: Die Freisetzungsgeschwindigkeit von Diclofenac aus einer Weichkapsel gemäss Beispiel 5
- Fig. 6: ein Diagramm mit den erfindungsgemäss geeigneten glasartigen Zuständen zwischen Ibuprofen K (hergestellt aus Titration von Ibuprofensäure und KOH), Ibuprofen und der Matrix Xylitol

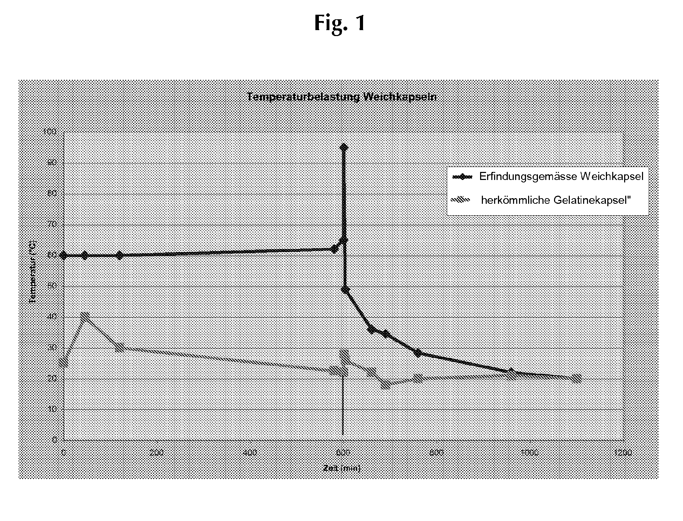

Aus Fig. 1 ist ersichtlich, dass bei der vorliegenden Erfindung ein deutlich höheres Temperaturprofil angelegt werden kann als im Fall herkömmlicher Weichkapseln aus Gelatine. Damit ist es möglich, auch Füllgüter mit höherem Schmelzpunkt (d.h. oberhalb von 35°C) in Weichkapseln bereitzustellen.

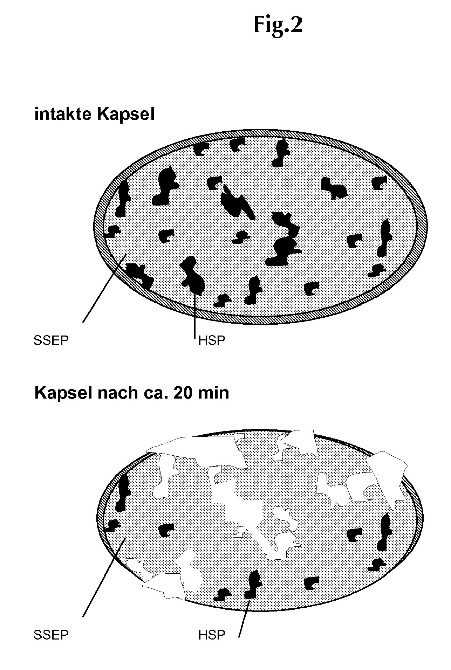

Fig. 2 zeigt eine bevorzugte Ausführungsform der erfindungsgemässen Weichkapsel. Hier umfasst das Füllgut eine vorstehend beschriebene selbstemulgierenden Phase (SSEP) sowie eine ebenfalls vorstehend beschriebene, zugemischte weitere feste hydrophile Phase (HSP). Die HSP bringt bei Gabe der Kapsel in ein wässriges Medium in einer Art Dochtwirkung Wasser in die Zubereitung. Dadurch wird der Zerfall der erfindungsgemässen Kapsel deutlich beschleunigt.

### Beispiel 1:

Ein gereinigter fraktionierter lipophiler Extrakt aus Pflanzen, insbesondere Soyabohne, enthaltend ca. 97% Sterol-Fettsäureester, (Vegapure® 95, Cognis) wurde über einen Schmelzbereich von ca. 25°C (visköse ölige Paste) bis 53° (klar geschmolzen) hinaus auf ca. 60°C erwärmt. Dosen von je 500 mg wurden in Weichkapseln mit einer Hülle aus Stärke, Sorbitol, Maltitol, Glycerin und Glycerinmonostearate (VegaGel™) gefüllt. Die Technik der Schmelzextrusionsherstellung des Kapselhüllenbandes wurde verwendet. Die Versiegelungstemperatur der Hüllennaht betrug 95°C. Die Kapseln ereichten Temperaturen von ca. 69°C, wurden anschliessend mit Luft von 20°C gekühlt und wiesen nach 3 Minuten eine Temperatur von weniger als 40°C aus. Die Kapseln waren lagerstabil und zerfielen in Wasser von 37°C innerhalb von 20 min. Das Füllgut schwamm auf und mischte sich nicht mit dem wässrigen Medium.

### Beispiel 2:

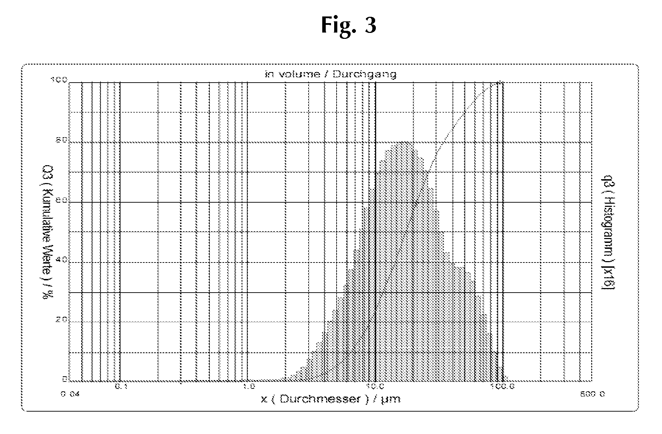

250 mg Vitamin E-Succinat (D-a-Tocopherol succinat, Smp. 76°C) wurden aufgeschmolzen und mit 70 mg Cremophor RH40 (Cognis, Polyoxyl-40-hydrogenated castor oil USP) und 30 mg MCT (Mittelkettige Triglyceride) bei 80°C unter Stickstoff intensiv gemischt. Je 350 mg der entstehenden leicht trüben Mischung mit einem Tropfpunkt von ca. 52-54°C wurden bei 60°C in Weichkapseln mit einer Hülle aus Polyvinylalkohol-stärke-acetal (PVACL, NatuteC™) mit Weichmacher Glycerin gefüllt. Die Technik der Schmelzextrusionsherstellung des Kapselhüllenbandes wurde verwendet. Die Versiegelungstemperatur der Hüllennaht betrug 130°C. Die Kapseln ereichten Temperaturen von ca. 85°C, wurden anschliessend mit Luft von 20°C gekühlt und wiesen nach 5 Minuten eine Temperatur von weniger als 40°C auf. Die Kapseln waren lagerstabil und zerfielen in Wasser von 37°C innerhalb von 45 min. Die Kapseln waren faktisch derart magensaftresistent, dass sie sich erst im Dünndarm öffnen würden. Das Füllgut schwamm zunächst auf. Das Vitamin E ging über eine Zeit von 2,5 h in eine Emulsion mit mittlerer Partikelgrösse ("Tröpfchengrösse") von 15-20 µm. Die erhaltene Tröpfchenverteilung ist in Fig. 3 gezeigt.

### Beispiel 3:

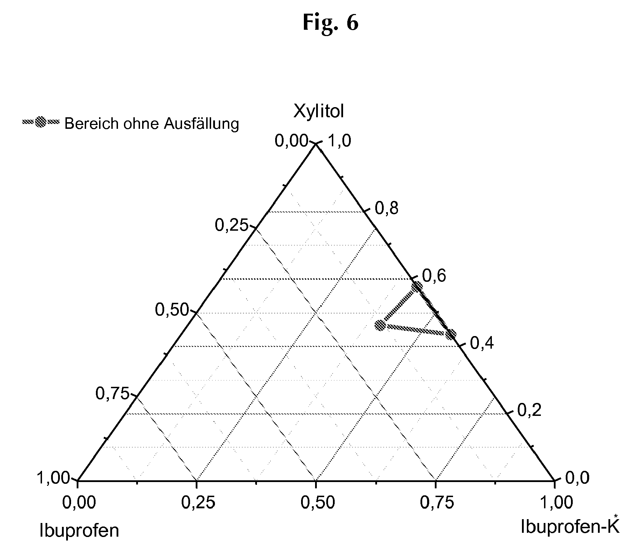

Eine kalte Mischung von 200 mg Xylitol (Smp. 93°C), 200mg Ibuprofen-Kaliumsalz, 24 mg Wasser und 24 mg PVP (Kollidon K12) wurden in einem 1-Wellenextruder homogen aufgeschmolzen und direkt einer Kapselbefüllung bei 90°C zugeführt. Im Rotary-Die Verfahren wurde für die 7.5 oval Kapsel mit Füllgewicht 448mg als Hüllenmaterial ein Polyvinylalkohol-Stärke-acetal (PVACL, Natutec^{™} verwendet. Die Siegeltemperatur betrug ca. 130°C. Die Kapsel wurde direkt nach der Herstellung in kaltes Paraffin (Decan) von ca. 0°C eingetragen. Der Kapselinhalt zeigte nach 6 Wochen noch keine Kristallisationserscheinungen. Beginnend 40 min mit Öffnung der Kapsel wurden innerhalb von 30 Minuten mehr als >90% des aktiven Wirkstoffs freigesetzt. In Fig. 6 ist ein Diagramm gezeigt, das die brauchbaren glasartigen Zustände zwischen Ibuprofen K (hergestellt aus Titration von Ibuprofensäure und KOH), Ibuprofen und der Matrix Xylitol darstellt.

### Beispiel 4:

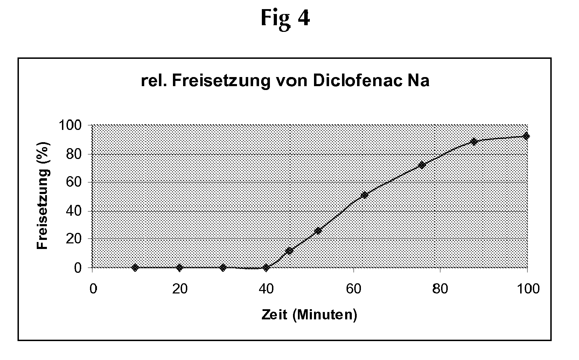

In eine Schmelze von 75mg Lutrol F127 (Poloxamer, Polyoxyethylen-polyoxypropylen Blockpolymer, BASF) (Smp. 53-57°C), 451 mg Gelucire 44/14 (PEG-32-glyceryl-laurate, Gattfossé (Smp. 44°C) wurden bei ca. 65°C 75mg Diclofenac Natrium (Smp. 283°C) feinkristallin eingetragen, und die Masse wurde bei 60°C in Dosen von je 601 mg in 11 minims oblong Weichkapseln mit einer Hülle aus Polyvinylalkohol-Stärke-acetal (PVACL, Natutec^{™}) gefüllt. Die Technik der Schmelzextrusionsherstellung des Kapselhüllenbandes wurde verwendet. Die Versiegelungstemperatur der Hüllennaht betrug 130°C. Die Kapseln erreichten Temperaturen von ca. 85°C, wurden mit Luft von 20°C gekühlt und wiesen nach 5 Minuten eine Temperatur von weniger als 40°C auf. Die Kapseln waren lagerstabil und zerfielen in Wasser von 37°C innerhalb von 45 min. Die Kapseln waren faktisch derart magensaftresistent, dass sie sich erst im Dünndarm öffnen würden. Diclofenac war nach einer Verzögerung von 45 min innerhalb von 1 Stunde zu rund 95% freigesetzt. Die entsprechenden Resultate sind in Fig. 4 gezeigt.

### Beispiel 5:

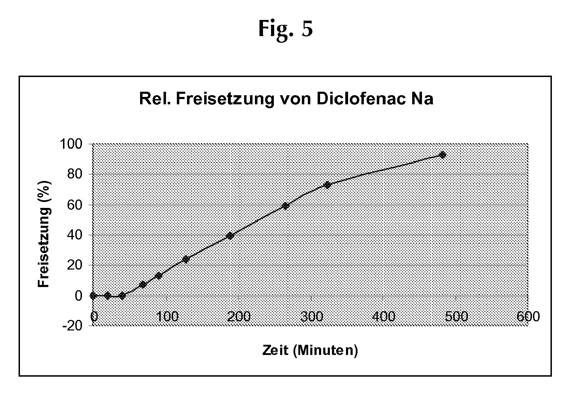

In eine Schmelze von 385 mg Lutrol F127 (Poloxamer, Polyoxyethylen-Polyoxypropylen-Blockpolymer, BASF) (Smp. 53-57°C), 385 mg Gelucire 50/13 (Stearoyl Macrogolglycerides, Gattfossé (Smp. 50°C) wurden bei ca. 65°C 75 mg Diclofenac Natrium (Smp. 283°C) feinkristallin eingetragen, und die Masse wurde bei 60°C in 14 minims oblong Weichkapseln mit einer Hülle aus Polyvinylalkohol-Stärke-acetal (PVACL, Natutec^{™}) gefüllt. Die Technik der Schmelzextrusionsherstellung des Kapselhüllenbandes wurde verwendet. Die Versiegelungstemperatur der Hüllennaht betrug 130°C. Die Kapseln erreichten Temperaturen von ca. 85°C, wurden mit Luft von 20°C gekühlt und wiesen nach 5 Minuten eine Temperatur von weniger als 40°C auf. Die Kapseln waren lagerstabil und zerfielen in Wasser von 37°C innerhalb von 45 min. Die Kapseln waren faktisch derart magensaftresistent, dass sie sich erst im Dünndarm öffnen würden. Diclofenac war nach einer Verzögerung von 45 min innerhalb von 7 Stunden zu rund 95% freigesetzt. Die entsprechenden Resultate sind in Fig. 5 gezeigt.

### Beispiel 6:

10 mg Temazepam wurden in einer Schmelze von 250 mg PEG4000 (mp53-58°C) bei 65°C gelöst. Zur Einstellung des richtigen Gleichgewichtsfeuchte (aw= 0.45) wurden der Masse 1.4 mg Wasser (0.55%) zugegeben und in Mengen von je 262 mg in Weichkapseln mit einer Hülle aus Stärke, Sorbitol, Maltitol, Glycerin und Glycerinmonostearate (Vega-Gel^{™}) gefüllt. Die Technik der Schmelzextrusionsherstellung des Kapselhüllenbandes wurde verwendet. Die Versiegelungstemperatur der Hüllennaht betrug 95°C. Die Kapseln erreichten Temperaturen von ca. 73°C, wurden anschliessend mit Luft von 20°C gekühlt und wiesen nach 3 Minuten eine Temperatur von weniger als 40°C auf. Die Kapseln waren lagerstabil und zerfielen in Wasser von 37°C innerhalb von 20 min. Das molekulardisperse Füllgut löste sich komplett innerhalb von 15 Minuten auf.

## Patentansprüche

1. Weichkapsel, umfassend eine Hülle und ein Füllgut mit mindestens einem pharmakologisch oder physiologisch aktiven Stoff, **dadurch gekennzeichnet, dass** die Hülle aus durch Schmelzextrusion thermoplastisch verarbeitbarem polymerem Material aufgebaut ist und das Füllgut unterhalb von 21°C fest ist und einen Tropfpunkt von höher als 37°C aufweist.

2. Weichkapsel nach Anspruch 1, **dadurch gekennzeichnet, dass** das Füllgut unterhalb von 30°C fest ist.

3. Weichkapsel nach Anspruch 1 oder 2, **dadurch gekennzeichnet, dass** das Füllgut einen Tropfpunkt von höher als 48°C, vorzugsweise höher 55°C aufweist.

4. Weichkapsel nach einem der vorhergehenden Ansprüche, **dadurch gekennzeichnet, dass** das durch Schmelzextrusion thermoplastisch verarbeitbare polymere Material ausgewählt ist aus der Gruppe bestehend aus Polyvinylpyrrolidon, Hydroxypropylcellulose, Hydroxypropylmethylcellulsose, Polyvinylalkohol, Polyvinylalkoholacetalen, Polethylenglycol-Polyvinylalkohol-Pfropfpolymer, Carbopol, Polymer aus Butylmethacrylat, 2-dimethylaminoethylmethacrylat und Methylmethacrylat im Verhältnis 1 : 2 : 1 , Polyethylacrylat, Methylmethacrylat, Polymethacrylsäure, Ethylacrylat, Polyethylacrylat, Methylmethacrylat, Trimethylammoniumethylmethacrylatchlorid, Hydroxypropylmethylcelluloseacetatsuccinat, Polyox, Stärke, Polymilchsäure, Polymilchsäure-coglycolid, Gelatine, Carrageenan, Casein, Gluten und deren Mischungen.

5. Weichkapsel nach einem der vorhergehenden Ansprüche, **dadurch gekennzeichnet, dass** das durch Schmelzextrusion thermoplastisch verarbeitbare polymere Material eine homogenisierte, Stärke enthaltende Masse ist, enthaltend vorzugsweise mindestens 45 Gew.-% einer amorphen Stärke mit einem Amylopektingehalt von grösser oder gleich 50 Gew.-% bezogen auf das Gewicht der wasserfreien Stärke, Wasser, mindestens einen organischen Weichmacher in einem Anteil von mindestens 12 Gew.-% bezogen auf das Gewicht der wasserfreien Stärke, wobei der Staudinger-Index der homogenisierten Masse mindestens 40 ml/g, bevorzugt mindestens 50 ml/g und noch bevorzugter mindestens 60 ml/g beträgt.

6. Weichkapsel nach einem der vorhergehenden Ansprüche, **dadurch gekennzeichnet, dass** das durch Schmelzextrusion thermoplastisch verarbeitbare polymere Material zusätzliche Stoffe enthält ausgewählt aus der Gruppe bestehend aus Weichmachern, Farbstoffen, UV-Stabilisatoren, Konservierungsstoffen, Antioxidantien, physikalisch und/oder chemisch modifizierten Biopolymeren, Zerfallsbeschleunigern, Formstabilisatoren, Gleit- und Entformmitteln und Trennmitteln.

7. Weichkapsel nach einem der vorhergehenden Ansprüche, **dadurch gekennzeichnet, dass** der pharmakologisch oder physiologisch aktive Stoff ein molekulardispers verteilter Wirkstoff ist.

8. Weichkapsel nach einem der vorhergehenden Ansprüche, **dadurch gekennzeichnet, dass** das Füllgut glasartig, teilkristallin oder kristallin ist.

9. Weichkapsel nach einem der vorhergehenden Ansprüche, **dadurch gekennzeichnet, dass** das Füllgut mindestens eine wirkstoffhaltige Schmelze und zusätzlich wirkstofftragende Partikel enthält.

10. Weichkapsel nach einem der vorhergehenden Ansprüche, **dadurch gekennzeichnet, dass** das Füllgut einen pharmakologisch oder physiologisch aktiven Stoff in einer festen lipophilen selbstemulgierenden Zubereitung umfasst.

11. Weichkapsel nach Anspruch 10, **dadurch gekennzeichnet, dass** das Füllgut eine weitere feste hydrophile Phase umfasst, welche bei Abfülltemperatur der Zubereitung in der selbstemulgierenden Phase selbst im wesentlichen unlöslich ist.

12. Verfahren zur Herstellung von Weichkapseln gemäss einem der Ansprüche 1 bis 11, umfassend die Schritte
a) Herstellung eines Hüllmaterials aus einem thermoplastisch verarbeitbaren polymerem Material durch Schmelzextrusion
b) Bereitstellung eines Füllguts durch derartiges Aufschmelzen, dass es pumpbar ist
c) Herstellung von Weichkapseln durch das Rotary-Die-Verfahren, wobei das aufgeschmolzene Füllgut in die Weichkapsel dosiert und die Kapsel anschliessend auf Umgebungstemperatur abgekühlt wird.

13. Verfahren nach Anspruch 12, **dadurch gekennzeichnet, dass** die Kapsel nach der Herstellung schockgekühlt wird, um Füllgut glasig zu machen.

14. Verfahren nach Anspruch 13, **dadurch gekennzeichnet, dass** die Schockkühlung durch kalte Gase oder ein durch kaltes Bad aus mit der Hülle verträglichen Flüssigkeiten erfolgt.

15. Verfahren nach einem der Ansprüche 12 bis 14, **dadurch gekennzeichnet, dass** das Füllgut bei tieferer Temperatur als der Verkapselungstemperatur durch einen das Füllgut isolierenden Füllkeil dosiert wird.

16. Verfahren nach einem der Ansprüche 12 bis 15, **dadurch gekennzeichnet, dass** das Füllgut unmittelbar vor der Verkapselung kontinuierlich aus den entsprechenden Ausgangskomponenten durch Schmelzen und Mischen hergestellt wird.

17. Verfahren nach einem der Ansprüche 12 bis 16, **dadurch gekennzeichnet, dass** mindestens ein Wirkstoff in fester Form kontinuierlich in die Füllgut-Schmelze dosiert und gelöst oder eingemahlen wird.

18. Verfahren nach einem der Ansprüche 12 bis 17, **dadurch gekennzeichnet, dass** der aktive Stoff in mikropartikulärer Form der Schmelze zugesetzt wird.

19. Verfahren nach einem der Ansprüche 12 bis 18, **dadurch gekennzeichnet, dass** zur kontinuierlichen Vorbereitung des Füllgutes ein Extruder verwendet wird.

20. Verwendung einer Kapsel gemäss einem der Ansprüche 1 bis 11 zur oralen, rektalen oder vaginalen Applikation.

21. Verwendung gemäss Anspruch 20, wobei die Kapsel als Nahrungsergänzungsmittel, Pharmazeutisches Produkt, Medizinal-Produkt oder für kosmetische oder technische Zwecke appliziert wird.
